Europäisches Patentamt

European Patent Office

Office européen des brevets

(11) Veröffentlichungsnummer: **0 553 351 B1**

## (12) EUROPÄISCHE PATENTSCHRIFT

(45) Veröffentlichungstag der Patentschrift: **27.12.95**

(21) Anmeldenummer: **91914958.3**

(22) Anmeldetag: **19.08.91**

(86) Internationale Anmeldenummer:
**PCT/SU91/00168**

(87) Internationale Veröffentlichungsnummer:
**WO 93/04034 (04.03.93 93/06)**

(51) Int. Cl.⁶: **C07C 227/38**, C07C 227/42,
C07C 229/60, A61K 9/08,
A61K 31/245, //(A61K31/245,
31:72)

---

(54) **Kristalline Modifikation des Hydrogenchlorids des 2-Dymethylaminoäthylesters der n-Butylamino-BenzoesÄure, Verfahren zur Herstellung dieser Modifikation und ArzneimittelprÄparat zur Anästhesie der Augen auf Ihrer Basis**

---

(43) Veröffentlichungstag der Anmeldung:
**04.08.93 Patentblatt 93/31**

(45) Bekanntmachung des Hinweises auf die
Patenterteilung:
**27.12.95 Patentblatt 95/52**

(84) Benannte Vertragsstaaten:
**AT BE CH DE DK FR GB IT LI NL SE**

(56) Entgegenhaltungen:

MIKROCHIMICA ACTA Bd. 1979 I, Nr. 3-4 ,
1979 , WIEN Seiten 207 - 219 M. KUHNERT-
BRANDSTÄTTER 'THERMOMIKROSKOPISCHE
DIREKTBESTIMMUNG DER KRISTALLISA-
TIONS- UND DER UMWANDLUNGSGE-
SCHWINDIGKEIT'

M.D. MASHKOVSKY; Lekarstvennye sredstva,
vol. I, 1986, Meditsina (Moscow), pages
325-328, 332-333.

(73) Patentinhaber: **LEONIDOV, Nikolai Borisovich**
**ulitsa Zatonnaya, 12, korpus 1, kv. 158**
**Moskau, 115407 (RU)**

(72) Erfinder: **LEONIDOV, Nikolai Borisovich**
**ul. Zatonnaya, 12-1-158**
**Moscow, 115407 (SU)**

(74) Vertreter: **Zellentin, Rüdiger et al**
**Zellentin & Partner,**
**Zweibrückenstrasse 15**
**D-80331 München (DE)**

---

MEZHDUNARODNAYA FARMAKOPEYA, vol. 3 "Spetsifikatsiya dlya kontrolya kachestva farmatsevticheskikh preparatov", 1990, voz (Geneva), pages 356-358.

M.V. RUBTSOV et al. "Sinteticheskie khimiko-farmatsevticheskie preparaty", 1971 Meditsina (Moscow), pages 74-77.

Thermochimica Acta vol. 153, 1989, Elsevier Science Publishers B.V. (Amsterdam), pages 11-26, R. CURINI et al. "Thermal analytical techniques applied to the narcotic field: cocaine analysis".

**Beschreibung**

Gebiet der Technik

Die vorliegende Erfindung bezieht sich auf des Gebiet der organischen Chemie, insbesondere auf eine neue kristalline Modifikation des Hydrogenchlorids des 2-Dimethylaminoethylesters der n-Butylaminobenzoesäure, ein Verfahren zur Herstellung dieser Modifikation und ein Arzneimittelpräparat zur Anästhesie der Augen auf ihrer Basis.

Zugrundeliegender Stand der Technik

Es ist eine kristalline Modifikation des Hydrogenchlorids des 2-Dimethylaminoethylesters der n-Butylaminobenzoesäure (Tetracain, Ametocain), polymorphe Form, bekannt (R.C.Sullivan, K.P.Obrien. X-ray diffraction studies of cocaine and its substitutes.-Bull. Narcotics, 1968, V. 20, N 3, p.31-40), die sich durch den folgenden Satz der Werte von Natzebenenabständen d und relativen Intensitäten von Reflexen I kennzeichnet:

| d, Å | I |
|------|-----|
| 12,55 | 100 |
| 8,38 | 42 |
| 6,28 | 12 |
| 5,42 | 8 |
| 5,05 | 67 |
| 4,83 | 12 |
| 4,43 | 8 |
| 4,22 | 20 |
| 3,97 | 11 |
| 3,78 | 10 |
| 3,56 | 8 |
| 3,48 | 17 |
| 3,30 | 10 |
| 3,12 | 10 |
| 2,96 | 6 |
| 2,805 | 7 |
| 2,661 | 5 |
| 2,507 | 5 |
| 2,151 | 4 |
| 1,949 | 4 |

Bekannt ist auch eine kristalline Modifikation des Hydrogenchlorids des 2-Dimethylaminoethylesters der n-Butylaminobenzoesäure (Ametocain, Tetracain), polymorphe Form (I.T.R.Owen, R.Sithiraks, F.A.Underwood, X-Ray Powder Diffraction Data for Seventeen Local Anesthetics. - I.Ass.Off Analyt. Chem., 1972, V.55), die sich durch den folgenden Satz der Werte von Netzebenenabständen d und relativen Reflexen I kennzeichnet:

| d, Å | I | d, Å | I |
|------|-----|------|----|
| 13,3 | 10 | 3,22 | 7 |
| 8,06 | 10 | 3,17 | 10 |
| 6,55 | 40 | 3,08 | 9 |
| 6,23 | 20 | 3,03 | 7 |
| 6,02 | 40 | 3,00 | 7 |
| 5,73 | 6 | 2,94 | 4 |
| 5,07 | 6 | 2,87 | 6 |
| 4,78 | 15 | 2,82 | 12 |
| 4,50 | 25 | 2,77 | 9 |
| 4,39 | 13 | 2,71 | 6 |
| 4,23 | 20 | 2,66 | 7 |
| 4,05 | 15 | 2,62 | 6 |
| 3,98 | 18 | 2,58 | 6 |
| 3,83 | 25 | 2,54 | 7 |
| 3,73 | 12 | 2,50 | 4 |
| 3,65 | 100 | 2,46 | 6 |
| 3,57 | 10 | 2,42 | 6 |
| 3,45 | 20 | 2,32 | 6 |
| 3,40 | 7 | 2,28 | 6 |
| 3,28 | 6 | 2,24 | 6 |
|      |    | 2,15 | 6 |

Die Schmelztemperatur der genannten Modifikationen liegt in den Grenzen zwischen 147 und 151 °C. Dabei stellt der Schmelzvorgang derselben eine Reihenfolge von zwei endothermen Effekten dar. (R.Curini, S.Zamponi, F.D'Ascenzo, S.De Angelis Curtis, A.Marino, A.Dezzi, Thermal analytical techniques applied to the narcotic field: cocaine analysis. - Thermochim.Acta, 1989, V. 153, N 1, p.11-26).

Es ist ein Verfahren zur Herstellung der genannten Modifikationen des Hydrogenchlorids des 2-dimethylaminoethylesters der n-Butylaminobenzoesäure bekannt, das den Prozess der n-Butylierung des $\beta$-Dimethylaminoethylesters der p-Aminobenzoesäure mit nachfolgender Kristallisation des erhaltenen Produktes im Gemisch mit Hydrogensulfit und Kohle aus dem Ethylalkohol bei 2 bis 3 °C und mit Trocknung einschliesst /Synthetische chemisch-pharmazeutische Präparate, Verlag "Meditsina" (Moskau), 1971, S. 75-77/.

Das gewonnene Produkt stellt die vorstehend beschriebenen kristallinen Modifikationen dar, die sich durch den besagten Satz der Werte d und I kennzeichnen. Die Schmelztemperatur der erhaltenen Verbindung liegt in den Grenzen zwischen 147 und 151 °C mit zwei aufeinanderfolgenden endothermen Effekten.

Das gewonnene Produkt stellt ein weisses Kristallpulver leicht bitteren Geschmacks dar, welches eine temporäre Anästhesie der Zunge hervorruft, wasser- und alkohollöslich ist, massig in Chloroform löslich und praktisch etherunlöslich ist.

Die bekannten kristallinen Modifikationen der genannten Verbindung zeigen eine lokalanästhetische Wirkung, besitzen aber dabei eine hohe Toxizität.

Überdies weisen die bekannten Modifikationen der genannten Verbindung eine bedeutende Nebenwirkung auf und rufen bei Verwendung von Arzneimittelpräparaten auf ihrer Basis in der Opthalmochirurgie Schwellungen der Lider, eine Beschädigung des Epithels der Kornea, das Auftreten von Erosionen, eine erhebliche Erweiterung der Bindehaut hervor. Augentropfen auf ihrer Basis kennzeichnen sich durch Lagerunbeständigkeit (die Haltbarkeitsdauer betragt 3 Monate unter der Bedingung einer zusätzlichen Stabilisierung der Lösung).

Die erfindungsgemässe kristalline Modifikation des Hydrogenchlorids des 2-Dimethylaminoethylesters der n-Butylaminobenzoesäure, das Verfahren zu ihrer Herstellung und die Verwendung sind neu und in der Literatur nicht beschrieben.

Offenbarung der Erfindung

Der Erfindung liegt die Aufgabe zugrunde, eine neue kristalline Modifikation des Hydrogenchlorids des 2-Dimethylaminoethylesters der n-Butylaminobenzoesäure zu schaffen, die eine hohe lokalanästhetische Wirkung besitzt und frei von Nebenwirkungen ist, sowie ein Verfahren zur Herstellung dieser Modifikation und ein Arzneimittelpräparat auf ihrer Basis zu entwickeln.

Diese Aufgabe wird dadurch gelöst, dass eine neue kristalline Modifikation des Hydrogenchlorids des 2-Dimethylaminoethylesters der n-Butylaminobenzoesäure vorgeschalagen wird. die sich duren nur einen endothermen Effekt des Schmelzens bei $1+8,6° \pm 0,3°C$ und durch folgende Werte von Netzehanenabständen d und relativen Intensitäten von Reflexen I kennzeichnet:

| I | d,Å | I | d,Å |
|---|---|---|---|
| 3 | 26,140 | 4 | 2,824 |
| 100 | 12,755 | 2 | 2,798 |
| 27 | 8,538 | 1 | 2,762 |
| 6 | 6,380 | 2 | 2,744 |
| 2 | 6,117 | 2 | 2,678 |
| 6 | 5,644 | 1 | 2,631 |
| 12 | 5,491 | 1 | 2,570 |
| 56 | 5,096 | 1 | 2,534 |
| 17 | 4,874 | 1 | 2,514 |
| 10 | 4,486 | 1 | 2,454 |
| 15 | 4,244 | 1 | 2,419 |
| 7 | 4,418 | 1 | 2,346 |
| 1 | 4,137 | 1 | 2,308 |
| 5 | 4,001 | 1 | 2,256 |
| 3 | 3,949 | 1 | 2,232 |
| 5 | 3,828 | 1 | 2,166 |
| 3 | 3,585 | 1 | 2,008 |
| 7 | 3,515 | 2 | 1,953 |
| 3 | 3,427 | 1 | 1,691 |
| 3 | 3,341 | 1 | |
| 3 | 3,310 | | |
| 3 | 3,170 | | |
| 4 | 3,156 | | |
| 4 | 3,149 | | |
| 1 | 3,064 | | |
| 2 | 2,979 | | |
| 1 | 2,944 | | |
| 1 | 2,903 | | |

Zur Erfindung gebört auch ein Verfahren zur Herstellung einer neuen kristallinen Modifikation der genannten Verbindung, welches erfindungsgemäss darin besteht, dass man eine Lösung des Hydrogenchlorids des 2-Dimethylaminoethylesters der n-Butylaminobenzoesäure polymorpher Form in Wasser oder in einem organischen Lösungsmittel oder in ihrem Gemisch durch ein Kältemittel mit einer Geschwindigkeit nicht unter 8°C/min bis zur vollständigen Kristallisation derselben unter nachfolgender Abtrennung der erhaltenen Kristalle und deren Trocknung abkühlt. Dabei ist als organisches Losungsmittel zweckmassigerweise Ethanol und als Kältemittel flüssiger Stickstoff zu verwenden. Die Trocknung führt man bevorzugt durch Vakuumieren bei einem Druck nicht höher als $10^{-2}$ Torr durch.

Die erhaltene neue kristalline Modifikation des Hydrogenchlorids des 2-Dimethylaminoethylesters der n-Butylaminobenzoesäure besitzt im Vergleich mit der bekannten polymorphen Form der genannten Verbindung eine erhöhte lokalanästhetische Aktivität und bedeutend verminderte Nebenwirkungen.

Die erfindungsgemässe neue kristalline Modifikation der genannten Verbindung findet Anwendung in der Opthalmologie.

Das Arzneimittelpräparat zur Anästhesie der Augen, welches einen wirksamen Stoff und ein pharmazeutisches Lösungsmittel enthält, beinhaltet erfindungsgemäss als wirksamen Stoff die anmeldungsgemässe kristalline Modifikation des Hydrogenchlorids des 2-Dimethylaminoethylesters der n-Butylaminobenzoesäure in einer Menge von 0,05 bis 5,0 Masse-%.

Zum Zwecke der effektivsten Benutzung des wirksamen Stoffes und Vergrösserung des prolongierten Effektes kann das erfindungsgemässe Präparat Alkylcellulose in einer Menge von 0,1 bis 0,75 Masse-% zusätzlich enthalten.

Als Alkylcellulose enthält das erfindungsgemässe Präparat bevorzugt wasserlösliche Methylcellulose.

5

Das erfindungsgemässe Präparat besitzt eine hohe lokalanästhetische Aktivität und ruft praktisch keine Nebenwirkungen hervor.

Die hohe lokalanästhetische Aktivität des wirksamen Stoffes gestattet es, seine Konzentration im erfindungsgemässen Arzneimittelapparat im Vergleich mit dem bekannten Präparat auf der Basis anderer Modifikationen (der polymorphen Form) um das Dreifache und mehr zu senken, was die Toxizität des Präparates bedeutend berabmindert. Das erfindungsgemässe Präparat kennzeichnet sich durch Lagerbeständigkeit, was seine Haltbarkeitsdauer zu erhöhen erlaubt (mehr als 1 Jahr).

Beste Ausführungsform der Erfindung

Die erfindungsgemässe neue kristalline Modifikation des Hydrogenchlorids des 2-Dimethylaminoethylesters der n-Butylaminobenzoesäure stellt ein feinstes Kristallpulver von weisser Farbe dar, das wasser- und alkohollöslich und praktisch etherunlöslich ist. Im Unterschied zu den bekannten kristallinen Modifikationen ist sie in Chloroform löslich. Eine 1%-ige wässrige Lösung der erfindungsgemässen Modifikation hat einen pH-Wert in den Grenzen zwischen 4,5 und 6,0.

Zum Unterschied von den bekannten polymorphen Formen kennzeichnet sich die erhaltene neue kristalline Modifikation durch nur einen endothermen Effekt des Schmelzens bei einer Temperatur von 148,6±0,3 °C.

Der Schmelzprozess der bekannten kristallinen Modifikationen der genannten Verbindung, die eine Schmelztemperatur in den Grenzen zwischen 147 und 151 °C haben, stellt die Reihenfolge von zwei endothermen Effekten dar.

Die gewonnenen thermoanalytischen Daten zeugan davon, dass der erfindungsgemässe Stoff eine neue kristalline Modifikation des Hydrogenchlorids des 2-Dimethylaminoethylesters der n-Butylaminobenzoesäure ist.

Zur Identifikation des erfindungsgemässen Stoffes wurde ein Komplex von chemischen, physikalisch-chemischen und Pharmakopöe-Analysemethoden angewandt.

Zur Bestätigung der neuen kristallinen Modifikation des erfindungsgemässen Stoffes wurde eine röntgenografische Phasenanalyse in der Guinier-Kammer mit der Registrierung von Beugungsmaxima auf dem Röntgenfilm angewendet.

Ein Vergleich der Warte von Netzebenenabständen d und relativen Intensitäten von Reflexen I des erfindungsgemässen Stoffes und der bekannten Modifikationen seigt , dass der erfindungsgemässe Stoff eine neue kristalline Modifikation des Hydrogenchlorids des 2-Dimethylaminoethylesters der n-Butylaminobenzoesäure ist.

Nach den Methodiken der qualitativen und quantitativen Bestimmung wurde festgestellt, dass der erfindungsgemässe Stoff das Hydrogenchlorid des 2-Dimethylaminoethylesters der n-Butylaminobenzoesäure mit der Reinheit 99,8% darstellt.

Diese Daten sind durch Methoden der hochauflösenden NMR$^{13}$C -Spektroskopie und der Gaschromatografie bestätigt.

Die Messungen der Parameter der hochauflösenden NMR$^{13}$C-Spektren wurden an einem NMR-Spektrometer mit supraleitendem Magnet mit einer Beobachtungsfrequenz für Kohlenstoffatome von 131,0 MHz vorgenommen. Die Messung der chemischen Verschiebungen NMR$^{13}$C - I$_H$ der Muster der bekannten Modifikation und der erfindungsgemässen Modifikation des genannten Stoffes wurde für gesättigt Lösungen derselben im DeuteroDimethylsulfoxid DMSO-D$_6$ mit der Genauigkeit 0.01 ppm durchgeführt. Die Werte der chemischen Verschiebungen NMR$^{13}$C wurden in Bezug auf das Signal des Losungsmittels gemessen und in die TMS-Skale (TMS-Si(CH$_3$)$_4$) umgerechnet:

$\delta$ $_{TMS}$ = $\delta_{DMSO}$ - 39,56 ppm. die Werte der chemischen Verschiebungen sind in der Tabelle 1 dargestellt. Eine Analyse der NMR$^{13}$C-Spektren der zu vergleichenden Muster der bekannten und der erfindungsgemässen Modifikation der genannten Verbindung hat gezeigt , dass in den Spektren keine Beimengungen auf dem Niveau des Signal-Rausch-Verhältnisses = 100 beobachtet wurden.

Die angeführten Daten lassen schlussfolgern, dass der neue Stoff gemäss der Erfindung eine chemische Formel hat, die der genannten Verbindung identisch ist.

Tabelle 1

Chemische Verschiebungen der Kohlenstoffatome $^{13}$C der bekannten und der erfindungsgemässen kristallinen Modifikation des Hydrogenchlorids des 2-Dimethylaminoethylesters der n-Butylaminobenzoesäure

| Lfd. Nr. | Stoff | Lage des Kohlenstoffatoms im Molekül der genannten Verbindung $C^1H_3-C^2H_2-C^3H_2-C^4H_2-NH$ (Benzolring) $C^9{=}O$ | | | | | |
|---|---|---|---|---|---|---|---|
| | | $C^1$ | $C^2$ | $C^3$ | $C^4$ | $C^5$ | $C^6$* |
| 1 | 2 | 3 | 4 | 5 | 6 | 7 | 8 |
| 1 | Bekannte Modifikation | 14,27 | 20,25 | 31,05 | 42,55 | 153,94 | 111,42 |
| 2 | Erfindungsgemässe Modifikation der genannten Verbindung | 13,75 | 19,76 | 30,37 | 41,94 | 153,31 | 110,74 |

Fortsetzung der Tabelle 1

| Lfd. Nr. | Lage des Kohlenstoffatoms im Molekül der genannten Verbindung $-C^{10}H_2 - C^{11}H_2 - (C^{12}H_3)_2 \cdot HCl$ | | | | | |
|---|---|---|---|---|---|---|
| | $C^7$* | $C^8$ | $C^9$ | $C^{10}$ | $C^{11}$ | $C^{12}$* |
| 1 | 9 | 10 | 11 | 12 | 13 | 14 |
| 1 | 132,19 | 115,06 | 166,37 | 58,80 | 56,10 | 43,46 |
| 2 | 131,48 | 114,63 | 165,63 | 58,30 | 55,08 | 42,53 |

* - die angeführten Werte entsprechen zwei äquivalenten Kohlenstoffatomen 13.

Die Messungen der Retentionszeit wurden an einer hocheffektiven Kapillarsäule mit unpolarer flüssiger Phase an einem Gaschromatograf durchgeführt.

Die Länge der Säule 30 m, die Geschwindigkeit des Trägergases (Helium) 40 cm/min. Die Messungen wurden bei Temperaturn 220, 230, 240 °C durchgeführt. Die in der Tabelle 2 angeführten Ergebnisse zeigen die Identität der Retentionszeiten der bekannten und der erfindungsgemässen kristallinen Modifikation der genannten Verbindung. Die angeführten Daten lassen schlussfolgern, dass die erfindungsgemässe kristalline Modifikation die chemische Formel der genannten Verbindung besitzt.

Tabelle 2

| Zeiten der geschromatografischen Retention der bekannten und der erfindungsgemässen kristallinen Modifikation des Hydrogenchlorids des 2-Dimethylaminoethylesters der n-Butylaminobenzoesäure | | | | |
|---|---|---|---|---|
| Nr. | Stoff | (220 °C) | (230 °C) | (240 °C) |
| 1 | Bekannte kristalline Modifikation | 15 12,0 | 11 21,1 | 8 42,9 |
| 2 | Erfindungsgemässe kristalline Modifikation | 15 11,6 | 11 21,3 | 8 43,0 |

Zum Vergleich der spektralen Kennlinien der bekannten kristallinen Modifikation und der erfindungsgemässen Modifikation der genannten Verbindung wurden ihre IR- und QR-Spektren gemessen.

Eine spektroskopische IR-Analyse wurde im Frequenzbereich von 400 bis 4000 cm$^{-1}$ in kVh durchgeführt.

IR-Kennfrequenzen sind in der Tabelle 3 angeführt.

Tabelle 3

IR-Kennfrequenzen der bekannten Modifikation und der erfindungsgemässen kristallinen Modifikation des Hydrogenchlorids des 2-Dimethylaminoethylesters der n-Butylaminobenzoesäure

| IR-Frequenzen, cm$^{-1}$ | |
|---|---|
| Bekannte Modifikation der genannten Verbindung | Erfindungsgemässe Modifikation der genannten Verbindung |
| 3368 | 3368 |
| 3000-2800 | 3000-2800 |
| 1688 | 1688 |
| 1600,1580,1536 | 1600,1580,1534 |
| 1480 | 1480 |
| 1470-1400 | 1470-1400 |
| 1400-1300 | 1400-1300 |
| 1286,1276 | 1284 |
| 1174, 1167 | 1172 |
| 1144 | 1144 |
| 1124,1120,1108 | 1124 |
| 1000-500 | 1000-500 |

Wie aus der Tabelle 3 folgt, besteht der Hauptunterschied des IR-Spektrums der erfindungsgemässen kristallinen Modifikation von der bekannten im Fehlen einer Aufspaltung der Banden bei 1284, 1172 und 1124 cm$^{-1}$.

QR-Spektren wurden im Frequenzbereich von 10 bis 2000 cm$^{-1}$ gemessen. QR-Kennfrequenzen sind in der Tabelle 4 angeführt.

8

Tabelle 3

IR-Kennfrequenzen der bekannten Modifikation und der erfindungsgemässen kristallinen Modifikation des Hydrogenchlorids des 2-Dimethylaminoethylesters der n-Butylaminoben-zoesäure

| IR-Frequenzen, cm$^{-1}$ | |
|---|---|
| Bekannte Modifikation der genannten Verbindung | Erfindungsgemässe Modifikation der genannten Verbindung |
| 3368 | 3368 |
| 3000-2800 | 3000-2800 |
| 1688 | 1688 |
| 1600,1580,1536 | 1600,1580,1534 |
| 1480 | 1480 |
| 1470-1400 | 1470-1400 |
| 1400-1300 | 1400-1300 |
| 1286,1276 | 1284 |
| 1174, 1167 | 1172 |
| 1144 | 1144 |
| 1124,1120,1108 | 1124 |
| 1000-500 | 1000-500 |

Die QR-Spektren der erfindungsgemässen Modifikation unterscheiden sich bedeutend von den QR-Spektren der bekannten Modifikation. Aus der Tabelle 4 ist ersichtlich, dass die bekannte Modifikation im Bereich 150-10cm$^{-1}$ ein hochstrukturiertes Spektrum hat, das aus acht Banden mit Maxima bei 122, 100, 87, 77, 62, 51, 37 und 23 cm$^{-1}$ besteht.

Die erfindungsgemässe neue kristalline Modifikation der genannten Verbindung kennzeichnet sich in diesem Bereich durch lediglich schwach ausgeprägte Banden bei 84, 58 und 39 cm$^{-1}$.

Somit zeugen die vorstehend angeführten Daten davon, dass der erfindungsgemässe Stoff eine neue kristalline Modifikation des Hydrogenchlorids des 2-Dimethylaminoethylesters der n-Butylaminobenzoesäure darstellt.

Es wurde die lokanästhetische Aktivität der erfindungsgemässen kristallinen Modifikation der genannten Verbindung im Vergleich mit der bekannten Modifikation (des in der Mikrochirurgie des Auges breit angewendeten Präparates Tetracain) studiert.

Dazu wurden eine 1%-ige Lösung des bekannten Präparates und 0,1-, 0,2-, 0,25- und 0,3%-ige Lösungen des erfindungsgemässen Stoffes in einer 0,9%-igen wässrigen Natriumchloridlösung zubereitet.

Die Untersuchungen wurden an 44 Tieren - an Chinchilla-Kaninchen - durchgeführt. Jede der aufgezählten Lösungen des genannten Stoffes wurde in gleicher Menge ins Auge des Kaninchens getröpfelt, wobei man die Zeit bis zum Eintritt der Anästhesie und deren Dauer nach der Methode der Berührung der Hornhaut des Auges vom Tier bestimmte. In allen Fällen wurden ins Auge je 0,06 ml der Lösung des Präparates (2 Tropfen zu je 0,03 ml) eingetröpfelt. 1,5 Stunden nach Aufhören der Anästhesie wurden die Kaninchen geschlachtet, das Versuchsauge wurde extierpiert und zur elektronischen Mikroskopie sowie für übliche histologische Untersuchungen abgegeben. Die Dauer der betäubenden Wirkung einer 0,1%-0,2%-, 0,25%- und 0,3%-igen Lösung des erfindungsgemässen Stoffes wurde mit der Dauer der betäubenden Wirkung eines genormten Anästhetikums - einer 1%-igen Lösung des bekannten Präparates - verglichen.

Experimentelle Werte der Zeit bis zum Eintritt der Anästhesie und die Abhängigkeiten der Dauer der betäubenden Wirkung sämtlicher geprüften Lösungen des Präparates von ihren Konzentrationen sind in der

Tabelle 5 ( $\alpha$ = 0,95) dargestellt.

Tabelle 5

| Nr. der Versuchsreihe | Präparat | Konzentration der Lösung, % | Anzahl der Tiere | Mittlere Zeit des Eintritts der Anästhesie, s | Mittlere Dauer der Anästhesie, min |
|---|---|---|---|---|---|
| 1 | 2 | 3 | 4 | 5 | 6 |
| 1 | Bekanntes Präparat<br>Erfindungsgemässer Stoff | 1<br>0,1 | 3<br>3 | 16,3±3,2<br>12,7±1,5 | 48,0±6,7<br>13,4±3,3 |
| 2 | Bekanntes Präparat<br>Erfindungsgemässer Stoff | 1,0<br>0,2 | 3<br>3 | 13,0±1,0<br>13,7±2,0 | 43,1±5,8<br>33,0±0,4 |
| 3 | Bekanntes Präparat<br>Erfindungsgemässer Stoff | 1,0<br>0,25 | 3<br>4 | 9,7±1,5<br>20,8±0,7 | 51,8±6,4<br>32,1±1,7 |
| 4 | Bekanntes Präparat<br>Erfindungsgemässer Stoff | 1,0<br>0,3 | 3<br>4 | 13,3±6,0<br>8,0±1,5 | 61,3±4,0<br>55,2±1,7 |

Es wurde der Einfluss der Operation der radialen Keratotomie auf die Dauer der betäubenden Wirkung der zu prüfenden Lösungen des genannten Stoffes untersucht. Die Werte der Zeit bis zum Eintritt der Anästhesie und die Dauer der Anästhesie, die durch Dicain-Lösungen bei der Durchführung der Operation der Keratotomie an Kaninchen hervorgerufen wird, sind in der Tabelle 6 ( $\alpha$ = 0,95) dargestellt.

Tabelle 6

| Nr. | Präparat | Konzentration der Lösung, % | Mittlere Zeit vor dem Eintritt der Anästhesie, s | Mittlere Dauer der Anästhesie, min |
|---|---|---|---|---|
| 1 | Bekanntes Präparat | 1,0 | 11,3±1,9 | 21,6±0,4 |
| 2 | Erfindungsgemässer Stoff | 0,3 | 11,3±0,9 | 15,7±0,5 |
| 3 | -"- | 0,25 | 11,0±0,6 | 12,4±0,3 |
| 4 | -"- | 0,1 | 15,0±0,7 | 6,9±0,5 |

Bei Verwendung einer 0,1%-igen Lösung des erfindungsgemässen Stoffes hat die Dauer der vollständigen Anästhesie es erlaubt, nur drei Etappen der aus fünf Etappen bestehenden Operation der Keratotomie durchzuführen, d.h. das Markieren, Anschnitte der Peripherie der Hornhaut und Anschnitte bis zur zentralen optischen Zone. Hiernach wurde die Empfindlichkeit des Auges gegen Operationsinstrumente wiederhergestellt. Bei Verwendung einer 0,25%-igen und besonders einer 0,3%-igen Lösung des erfindungsgemässen Stoffes war die Anästhesiedauer hinreichend für die Durchführung aller fünf Etappen der Operation der Keratotomie, die 3 min dauert (das heisst auch der Kontrolle der Tiefe und Vertiefung des Anschnittes und des Stadiums der Spülung des Anschnittes). Bei Verwendung einer 0,25%-igen und einer 0,3%-igen Lösung des erfindungsgemässen Stoffes war das Auge des Kaninchens gegen chirurgische Manipulationen vollends unempfindlich. Eine 0,3-ige Lösung des erfindungsgemässen Stoffes hat im Vergleich mit einer 1%-igen Lösung des bekannten Präparates während der Operation eine ähnliche Tiefe der Anästhesie sichergestellt, die für die Durchführung aller Operationsetappen ausreichend ist.

Bemerkenswert ist, dass der Operation seingriff die Dauer der Anästhesie sowohl bei Verwendung der Lösungen des erfindungsgemässen Stoffes sämtlicher untersuchten Konzentrationen als auch bei Verwendung einer 1%-igen Lösung des bekannten Präparates verkürzt. Jedoch erfolgt die Reduzierung der Dauer der betäubenden Wirkung der Lösung der erfindungsgemässen Stoffes und der Lösung des bekannten Präparates in verschiedenem Grade: bei Verwendung einer 0,1%-gen Lösung des erfindungsgemässen Stoffes nimmt die Dauer der betäubenden Wirkung im Mittel von 13 min auf 7 min (46,2%) ab; im Falle einer 0,25%-igen Lösung - von 32 min auf 12 min (62,5%); im Falle einer 0,3%-igen Losung von 55 min auf 16 min (70,9%); im Falle einer 1%-igen Lösung des bekannten Präparates von 51 min auf 22 min (56,9%).

Beim Tröpfeln einer 1%-igen Lösung des bekannten Präparates ins Auge eines Kaninchens wird die Oberfläche des Auges wellig und matt. Aber bei der Einwirkung der Lösungen des erfindungsgemässen Stoffes sämtlicher untersuchten Konzentrationen auf das Auge eines Kaninchens wurden keine Veränderungen der Oberfläche des Auges beobachtet; visuell unterscheidet sich das untersuchte Auge nicht vom intakten.

Im Ergebnis der durchgeführten Teste kann man schlussfolgern, dass die Dauer der betäubenden Wirkung einer 0,3%-igen Lösung des erfindungsgemässen Stoffes und einer 1%-igen Lösung des bekannten Präparates, das in der ophthalmoligischen chirurgischen Praxis eingesetzt wird, ungefähr gleich ist und im Mittel 55 min beträgt (der Unterschied 55,2±1,7 min und 61,3 ± 4,0 min ist unglaubwürdig). Bei ähnlicher Tiefe der Anästhesie, die durch eine 0,3%-ige Lösung des erfindungsgemässen Stoffes und eine 1%-ige Lösung des bekannten Präparates hervorgerufen wird, tritt die Wiederherstellung der Empfindlichkeit der Augen bei Verwendung der Lösung des erfindungsgemässen Stoffes früher als bei Verwendung der Lösung des bekannten Präparates ein. Eine 0,3%-ige Losung des erfindungsgemässen Stoffes, die nach Tiefe una Dauer der betaubenden Wirkung mit einer 1%-igen Losung des bekannten Praparates gleich ist, ruft zum Unterschied von dem letzteren keine Welligkeit und Mattheit der Oberfläche des Auges eines Kaninchens hervor. Nach dem Eintröpfeln der Lösungen des erfindungsgemässen Stoffes sämtlicher untersuchten Konzentrationen unterscheidet sich das Auge visuell nicht von einem intakten. 0,1%-, 0,2%- und 0,25%-ige Lösungen des erfindungsgemässen Stoffes können zur lokalen Anästhesie des Auges bei verschiedenen kurzzeitigen schmerzhaften Manipulationen und Prozeduren verwendet werden.

Somit zeugen die angeführten Daten davon, dass die erfindungsgemässe kristalline Modifikation des genannten Stoffes eine erhöhte lokalanästhetische Aktivität und verminderte Nebenwirkungen zeigt. Die genannten Eigenschaften gestatten es, die Dosierung des Präparates bei Erhöhung seiner therapeutischen Effektivität erheblich zu reduzieren.

Die erfindungsgemässe kristalline Modifikation der genannten Verbindung ist wirksamer Stoff eines Arzneimittelpräparates zur Anästhesie der Augen.

Das erfindungsgemässe Arzneimittelpräparat enthält den wirksamen Stoff in einer Menge von 0,05 bis 0,5 Masse-% und ein beliebiges, für Augentropfen geeignetes pharmazeutisches Lösungsmittel. Zusätzlich kann es ein beliebiges Derivat der Alkylcellulose in einer Menge von 0,1 bis 0,75 Masse-%, im besonderen wasserlösliche Methylcellulose enthalten.

Die Wahl der Konzentration des wirksamen Stoffes ist dadurch bedingt, dass im genannten Bereich der Konzentrationen ein hoher lokalanästhetischer Effekt bei Fehlen von Nebenwirkungen in Erscheinung tritt.

Das erfindungsgemässe Arzneimittelpräparat kann ein beliebiges Cellulosealkylderivat (Methyl, Ethyl, Propyl u.a.) enthatlten, da sie alle eine Erhöhung der Viskosität der Tropfen bewirken, was zur effektivsten Ausnutzung des wirksamen Stoffes beiträgt.

Die Wahl der Alkylcellulose in dem erfindungsgemässen Arzneimittelpräparat ist dadurch bedingt, dass im genannten Bereich eine solche Viskosität des Arzneimittelpräparates erreicht wird, bei welcher dessen beste Haftung am Gewebe des Augapfels stattfindet. Bei einer Konzentration der Alkylcellulose unter 0,1% ist die Viskosität des erfindungsgemassen Präparates derart gering, dass die Tropfen beim Eintröpfeln ins Auge frei unter dem Lid hervorfliessen.

Bei einer Konzentration der Alkylcellulose über 0,75% steigt die Viskosität dermassen, dass das erfindungsgemässe Arzneimittelpräparat über das Augapfel unmöglich gleichmässig verteilt werden kann.

Die bevorzugte Verwendung der Methylcellulose ist dadurch hervorgerufen, dass sie mit dem wirksamen Stoff am besten vereinbar ist, den höchsten Effekt seiner Freisetzung aus dem Arzneimittelpräparat ergibt und in der medizinischen Praxis breit angewendet wird.

Untersuchungen der lokalanästhetischen Aktivität des erfindungsgemässen Arzneimittelpräparates wurde im Vergleich mit dem bekannten Präparat an Chnchilla-Kaninchen durchgeführt. Jede von den zubereiteten Lösungen tröpfelte man in gleicher Menge in das Auge eines Kaninchens ein und bestimmte die Zeit bis zum Eintritt der Anästhesie und deren Dauer nach der Methode der Berührung der Hornhaut des Auges vom Tier (Renier-Methode). In allen Fällen tröpfelte man ins Auge je 0,06 ml der Lösung des Präparates (2 Tropfen zu je 0,03 ml) ein.1,5 Stunden nach Aufhören der Anästhesie wurden die Kaninchen geschlachtet, das Auge wurde exstierpiert und es wurden hystologische Untersuchungen vorgenommen.

Daten der experimentellen Untersuchungen sind in der Tabelle 7 dargestellt ($\alpha = 0,95$).

Tabelle 7

| Nr. der Versuche | Zusammensetzung des Arzneimittelpräparates, Masse-% | Mittlere Zeit des Eintritts der Anästhesie, s | Mittlere Dauer der Anästhesie, min | Nebenwirkungen |
|---|---|---|---|---|
| 1 | 2 | 3 | 4 | 5 |
| 1 | Bekanntes Präparat: wirksamer Stoff 0,25, Natriumchlorid 0,9, destilliertes Wasser bis zu 100 | $13,4^{\pm}2,0$ | $15,3^{\pm}2,0$ | Die Oberfläche des Auges wird matt |
| 2 | Bekanntes Präparat: wirksamer Stoff 0,5, Natriumchlorid 0,9, destilliertes Wasser bis zu 100 | $13,0^{\pm}1,0$ | $26,4^{\pm}3,2$ | Die Oberfläche des Auges wird wellig und matt |
| 3 | Bekanntes Präparat: wirksamer Stoff 1,0, Natriumchlorid 0,9, destilliertes Wasser bis zu 100 | $13,^{\pm}1,0$ | $48,0^{\pm}3,3$ | Welligkeit und Mattheit nehmen zu |

| 1 | 2 | 3 | 4 | 5 |
|---|---|---|---|---|
| 4 | Erfindungsgemässes Präparat: wirksamer Stoff 0,05, Natriumchlorid 0,9, destilliertes Wasser bis zu 100 | $15,0 \pm 2,0$ | $3,2 \pm 1,5$ | nicht festgestellt |
| 5 | Erfindungsgemässes Präparat: wirksamer Stoff 0,1, Natriumchlorid 0,9, destilliertes Wasser bis zu 100 | $12,7 \pm 1,5$ | $13,4 \pm 3,3$ | Nicht festgestellt |
| 6 | Erfindungsgemässes Präparat: wirksamer Stoff 0,3, Natriumchlorid 0,9, destilliertes Wasser bis zu 100 | $3,0 \pm 1,5$ | $55,2 \pm 1,7$ | Nicht festgestellt |
| 7 | Erfindungsgemässes Präparat: wirksamer Stoff 0,3, Natriumchlorid 0,9, Methylcellulose 0,1, destilliertes Wasser bis zu 100 | $5,3 \pm 1,0$ | $75,2 \pm 3,0$ | Nicht festgestellt |

| 1 | 2 | 3 | 4 | 5 |
|---|---|---|---|---|
| 8 | Erfindungs- gemässes Präpa- rat: wirksamer Stoff 0,3, Natriumchlorid 0,9, Methyl- cellulose 0,5, destilliertes Wasser bis zu 100 | $5,0^{\pm}1,0$ | $75,7^{\pm}3,5$ | Nicht festge- stellt |
| 9 | Erfindungs- gemässes Präpa- rat: wirksamer Stoff 0,3, Natriumchlorid 0,9, Methyl- cellulose 0,75, destilliertes Wasser bis zu 100 | $4,5^{\pm}0,5$ | $69,3^{\pm}2,1$ | Nicht festge- stellt |

Aus der Tabelle folgt, dass das erfindungsgemässe Arzneimittelpräparat, das eine hohe anästhesierende Aktivität besitzt, praktisch keine Nebenwirkungen hervorruft. Die hohe lokalanästhetische Aktivität des wirksamen Stoffes des erfindungsgemässen Präparates gestattet es, seine Konzentration im Arzneimittelapparat gegenüber dem bekannten Präparat um das Dreifache und mehr zu vermindern und somit die Toxizität des Präparates beträchtlich herabzusetzen.

Das erfindungsgemässe Präparat wurde in einer Klinik an Menschen erprobt. Die Teste wurden bei der Operation der radialen Keratotomie durchgeführt. Es wurden drei Varianten von Arzneimittelpräparaten angewendet: eine 1%-ige Lösung des bekannten Präparates (auf Basis der bekannten Modifikation des Hydrogenchlorids des 2-Dimethylaminoethylesters der n-Butylaminobenzoesäure) (Variante 1); eine 0,25%- und eine 0,3%-ige Lösung des erfindungsgemässen Präparates (Varianten 2 und 3) bei der Operation der radialen Keratotomie. Die Lösung der Variante 1 tröpfelte man in jeder der ersten drei Etappen der Operation. Bei allen Kranken ohne Ausnahme wurde ein ausgeprägtes Ödem der Kornea, die Desquamation des Epithels während der Operation beobachtet, das Ödem erhielt sich bei zu 2 Tagen auch nach der Operation.

Die Losung der Variante 2 wurde für die Oberflächenanästhesie des Auges bei 28 Kranken verwendet. Diese Lösung tröpfelte man nur einmal ein- in der ersten Etappe der Operation, jedoch erwies sich die Tiefe der Narkose für die Durchführung der Operation bei einem Teil der Kranken als unzureichend.

Die Lösung der Variante 3 wurde für die Oberflächenanästhesie des Auges bei 80 Kranken verwendet. Die Lösung tröpfelte man ebenfalls nur einmal in der ersten Etappe der Operation ein. Die Anästhesie erwies sich für die Durchführung der genannten Operation als ausreichend. Bei 5 Kranken (6,2%) wurden Resterscheinungen der Berührungsempfindlichkeit ohne Schmerzempfindungen beobachtet, welche die Operation nicht behinderten. Für die Durchführung der Operation bei einem Kranken verwendete man im Mittel 5 ml einer 1%-igen Lösung des bekannten Präparates und nur 0,1 ml einer 0,25%- und einer 0,3%-igen Lösung des erfindungsgemässen Präparats.

Im Ergebnis der durchgeführten Untersuchungen wurde festgestellt, dass die betäubende Wirkung einer 0,3%-igen Lösung des erfindungsgemässen Präparates bei der 50-fachen Verringerung seines Volumens beim Durchführein der Operation der Keratotomie der betäubenden Wirkung einer 1%-igen Lösung des bekannten Präparates entspricht. Bei Verwendung der genannten Lösung des erfindungsgemässen Präparates fehlt zum Unterschied von dem verwendeten bekannten Präparat ein Ödem der Kornea bei allen Kranken.

Das erfindungsgemässe Arzneimittelpräparat zur Anästhesie der Augen stellt eine durchsichtige farblose Flüssigkeit mit einem pH-Wert von 4,3 bis 6,8 dar.

14

Man bereitet das erfindungsgemässe Präparat nach einer bekannten Methodik zu.

Das erfindungsgemässe Präparat bewahrt man bei der Temperatur 25°C an einer lichtgeschützten Stelle auf. Die Aufbewahrungszeit beträgt 12 Monate.

Es wurde eine Tauglichkeitsanalyse des erfindungsgemässen Präparates nach 6 und 12 Monaten der Aufbewahrung durchgeführt.

Die Ergebnisse der Analyse zeugen davon, dass das erfindungsgemässe Präparate nach 12 Monaten der Aufbewahrung seine Hauptkennziffern nicht verändert, weshalb man über die Tauglichkeit des genannten Präparates schlussfolgern kann.

Das erfindungsgemässe Arzneimittelpräparat zur Anästhesie der Augen erlaubt es, die Anzahl der Komplikationen der Nachoperationsperiode durch Eliminierung von Nebenwirkungen zu verringern, die Toxizität durch Senkung der Konzentration des wirksamen Stoffes im Präparat unter Beibehaltung des lokalanästhetischen Effektes zu vermindern und die Haltbarkeitsdauer bis zu 12 Monaten zu verlängern (die Haltbarkeitsdauer des bekannten Präparates beträgt 3 Monate).

Gemäss der Erfindung erhält man den wirksamen Stoff des anmeldungsgemässen Präparates - die neue kristalline Modifikation des Hydrogenchlorids des 2-Dimethylaminoethylesters der n-Butylaminobenzoesäure - durch Kühlung einer Lösung polymorpher Form der genannten Verbindung in Wasser oder einem organischen Lösungsmittel oder in deren Gemisch durch ein Kältemittel mit einer Geschwindigkeit nicht unter 8°C/min bis zur vollständigen Kristallisation derselben unter nachfolgender Abtrennung der erhaltenen Kristalle und deren Trocknung.

Als Kältemittel kann ein beliebiger Stoff eingesetzt werden, der die Temperatur des zu kühlenden Stoffes mit einer Geschwindigkeit nicht unter 8°C/min zu senken vermag. Der optimalste Stoff, der als Kältemittel zum Einsatz kommt, ist flüssiger Stickstoff, dessen Verwendung es gestattet, die Ausbeute an Zielprodukt durch schnelle Einstellung und weitere Aufrechterhaltung der erforderlichen Kühlgeschwindigkeit zu erhöhen.

Die Wahl der Geschwindigkeit der Kühlung der Ausgangslösung des genannten Stoffes durch das Kältemittel erklärt sich dadurch, dass das gesteckte Ziel (Herstellung einer neuen kristallinen Modifikation) bei einer Kühlgeschwindigkeit nicht unter 8°C/min erreicht wird. Die Durchführung des Kristallisationsprozesses bei einer Kühlgeschwindigkeit unter 8°C/min gestattet es nicht, die neue kristalline Modifikation nach der Erfindung zu erhalten.

Die obere Grenze der Kühlgeschwindigkeit ist nicht beschränkt. Bei einer beliebigen maximal erreichbaren Kühlgeschwindigkeit der Ausgangslösung findet die Bildung der neuen kristallinen Modifikation statt.

Den Kühlvorgang bringt man in Wasser oder in einem beliebigen organischen Lösungsmittel oder in deren Gemisch zustande, in denen der Ausgangsstoff löslich ist. Zu den optimalen Lösungsmitteln, in denen der Ausgangsstoff gut löslich ist, gehören Wasser und Äthanol. Dabei wird die grösste Zielproduktausbeute erreicht.

Der erfindungsgemässe Stoff lässt sich unabhängig von der Konzentration des Ausgangsstoffes in der Lösung erhalten.

Die Wahl des Trocknungsverfahrens bei einem Druck von höchstens $10^{-2}$ Torr ist dadurch bedingt, dass das ausgetrocknete Fertigprodukt eine Feuchtigkeit nicht über 3% aufweisen soll.

Die Trocknung bei einem Druck höher als $10^{-2}$ Torr führt zur Herstellung einer auseinanderfliessenden instabilen Rohmasse.

Zum besseren Verständnis der vorliegenden Erfindung werden Beispiele für die Herstellung des erfindungsgemässen Stoffes angeführt.

Beispiel 1

500 ml einer 10%-igen wässrigen Lösung des Hydrogenchlorids des 2-Dimethylaminoethylesters der n-Butylaminobenzoesäure (polymorpher Form) kühlt man durch flüssigen Stickstoff mit der Geschwindigkeit 8°C/min bis zur vollständigen Kristallisation derselben. Die erhaltene gefrorene Masse trägt man auf Untersätze auf und bringt in einen Sublimator ein. Die Trocknung führt man bei dem Druck $10^{-2}$ Torr bis zur Restfeuchte von 3% durch. Die Zielproduktausbeute beträgt 95,7 Masse-%.

Der gewonnene Stoff kennzeichnet sich durch Werte von Netzebenenabständen d und relativen Reflexen I, die mit den entsprechenden obengenannten Werten der neuen kristallinen Modifikation des Hydrogenchlorids des 2-Dimethylaminoethylesters der n-Aminobenzoesäute zusammenfallen, hat eine Schmelztemperatur von 148,6±0,3°C mit nur einem endothermen Effekt. Nach allen Kennwerten (NMR, IR-, QR-Spektren) entspricht der gewonnene Stoff der erfindungsgemässen neuen kristallinen Modifikation der genannten Verbindung.

15

Beispiel 2

Den Prozess führt man ähnlich wie im Beispiel 1 beschrieben durch, wobei die Konzentration des Ausgangsstoffes in Wasser 50 Masse-% beträgt.

Die Zielproduktausbeute 96,2 Masse-%.

Der gewonnene Stoff mit einer Schmelztemperatur von 148,6± 0,3°C mit nur einem endothermen Effekt hat Charakteristiken, die zu dem Beispiel 1 analog sind.

Beispiel 3

Den Prozess führt man ähnlich wie im Beispiel 1 beschrieben durch, wobei die Kühlgeschwindigkeit gleich 300°C/min ist.

Die Zielproduktausbeute 97,5 Masse-%.

Man erhält einen Stoff mit einer Schmelztemperatur von 148,6±0,3°C mit nur einem endothermen Effekt, welcher Stoff Charakterisktiken hat, die zu dem Beispiel 1 analog sind.

Beispiel 4

Den Prozess führt man ähnlich wie im Beispiel 1 beschrieben durch, wobei die Trocknung in einem Sublimator bei dem Druck $10^{-5}$ Torr erfolgt.

Die Zielproduktausbeute 96,8 Masse-%.

Der gewonnene Stoff hat Charakteristiken, die zu dem Beispiel 1 analog sind.

Beispiel 5

500 ml einer 10%-igen Lösung des Hydrogenchlorids des 2-Dimethylaminoethylestersder n-Butylaminobenzoesäure (polymerpher Form) in Athanol kühlt man durch flüssigen Stickstoff mit der Geschwindigkeit 8°C/min bis zur vollständigen Kristallisation derselben. Die erhaltene gefrorene Masse trägt man auf Untersätze auf und bringt diese in einen Sublimator ein. Die Trocknung führt man bei dem Druck $10^{-2}$ Torr durch.

Die Zielproduktausbeute 95,6 Masse-%.

Der gewonnene Stoff entspricht nach allen Parametern dem Stoff gemäss dem Beispiel 1 mit einer Schmelztemperatur von 148±0,3°C mit nur einem endothermen Effekt.

Beispiel 6

Den Prozess führt man ähnlich dem Beispiel 5 durch, wobei die Konzentration des Ausgangsstoffes in Ethanol 50 Masse-% beträgt.

Die Zielproduktausbeute 97,0 Masse-%.

Der gewonnene Stoff hat Charakteristiken, die zu dem Beispiel 1 analog sind.

Beispiel 7

Den Prozess führt man ähnlich wie im Beispiel 5 beschrieben mit der Kühlgeschwindigkeit 300°C/min durch.

Die Zielproduktausbeute 98,2 Masse-%.

Der gewonnene Stoff hat Charakteristiken, die zu dem Beispeil 1 analog sind.

Beispiel 8

Den Prozess führt man ähnlich wie im Beispiel 5 beschrieben durch, wobei die Trocknung in einem Sublimator bei dem Druck $10^{-5}$ Torr erfolgt. Die Zielproduktausbeute 96,3 Masse-%.

Der gewonnene Stoff hat Charakteristiken, die zu dem Beispiel 1 analog sind.

Beispiel 9

Man nimmt 500 ml einer 10%-igen Lösung des Ausgangsstoffes im Wasser-Athanol-Gemische (1:1). Den Prozess führt man ähnlich wie im Beispiel 5 beschrieben durch. Die Zielproduktausbeute 96,0 Masse-

%.

Der gewonnene Stoff hat Charakteristiken, die zu dem Beispiel 1 analog sind.

Gewerbliche Verwertbarkeit

Die erfindungsgemässe kristalline Modifikation des Hydrogenchlorids des 2-Dimethylaminoethylesters der n-Butylaminobenzoesäure besitzt eine lokalanästhetische Wirkung und kann auf verschiedenen Gebieten der Medizin breite Anwendung finden, im besonderen kommt ein Arzneimittelpräparat zur Anästhesie der Augen auf Basis des erfindungsgemässen Stoffes in der Ophthalmologie bei chirurgischen Operationen zur Verwendung.

**Patentansprüche**

1. Kristalline Modifikation des Hydrogenchlorids des 2-Dimethylaminoethylesters der n-Butylaminobenzoesäure,
   **gekennzeichnet** durch nur einen endothermen Effekt des Schmelzens bei 148,6±0,3 °C und folgende Netzebenenabstände und relative Intensität von Reflexen I:

| I | d,Å | I | d,Å |
|---|-----|---|-----|
| 3 | 26,140 | 4 | 2,824 |
| 100 | 12,755 | 2 | 2,798 |
| 27 | 8,538 | 1 | 2,762 |
| 6 | 6,380 | 2 | 2,744 |
| 2 | 6,117 | 2 | 2,678 |
| 6 | 5,644 | 1 | 2,631 |
| 12 | 5,491 | 1 | 2,570 |
| 56 | 5,096 | 1 | 2,534 |
| 17 | 4,874 | 1 | 2,514 |
| 10 | 4,486 | 1 | 2,454 |
| 15 | 4,244 | 1 | 2,419 |
| 7 | 4,418 | 1 | 2,346 |
| 1 | 4,137 | 1 | 2,308 |
| 5 | 4,001 | 1 | 2,256 |
| 3 | 3,949 | 1 | 2,232 |
| 5 | 3,828 | 1 | 2,166 |
| 3 | 3,585 | 1 | 2,008 |
| 7 | 3,515 | 2 | 1,953 |
| 3 | 3,427 | 1 | 1,691 |
| 3 | 3,341 | 1 | |
| 3 | 3,310 | | |
| 3 | 3,170 | | |
| 4 | 3,156 | | |
| 4 | 3,149 | | |
| 1 | 3,064 | | |
| 2 | 2,979 | | |
| 1 | 2,944 | | |
| 1 | 2,903 | | |

2. Verfahren zur Herstellung einer kristallinen Modifikation des Hydrogenchlorids des 2-Diemethylaminoethylesters der n-Butylaminobenzoesäure nach Anspruch 1, dadurch **gekennzeichnet,** dass man eine Lösung des Hydrogenchlorids des 2-Dimethylaminoethylesters der n-Butyl-aminobenzoesäure polymorpher Form in Wasser oder einem organischen Lösungsmittel oder in ihrem Gemisch durch ein Maltemittel mit einer Geschwindigkeit nicht unter 8 °C/min bis zur vollständigen Kristallisation derselben unter nachfolgender Abtrennung der erhaltenen Kristalle und deren Trocknung abkühlt.

3. Verfahren nach Anspruch 2, dadurch **gekennzeichnet**, dass man als orgainsches Lösungsmittel Ethanol verwendet.

4. Verfahren nach Anspruch 2, dadurch **gekennzeichnet,** dass man als Kältemittel flüssigen Stickstoff verwendet, während man die Trocknung durch Vakuumieren bei einem Druck von höchstens $10^{-2}$ Torr vornimmt.

5. Arzneimittelpräparat zur Anästhesie der Augen, das einen wirksamen Stoff und ein pharmazeutisches Lösungsmittel enthalt, dadurch **gekennzeichnet**, dass es als wirksamen Stoff die kristalline Modifikation des Hydrogenchlorids des 2-Dimethylaminoethylesters der n-Butylaminobenzoesäure nach Anspruch 1 in einer Menge von 0,05 bis 0,5 Masse-% enthält.

6. Arzeuimittelpräparat nach Anspruch 5, dadurch **gekennzeichnet,** dass es Alkylcellulose in einer Menge von 0,1 bis 0,75 Masse-% zusätzlich enthält.

7. Arzneimittelpräparat nach Anspruch 6, dadurch **gekennzeichnet,** dass es als Alkylzellulose wasserlosliche Methylcellulose enthalt.

## Claims

1. Crystalline modification of the hydrogen chloride of the 2-dimethylaminoethyl ester of n-butylaminobenzoic acid, characterized by only one endothermic effect of the melt at 148.6 ± 0.3 °C and the following interplanar spacings and relative intensity of reflections I:

| I | d,Å | I | d,Å |
|-----|--------|---|-------|
| 3 | 26,140 | 4 | 2,824 |
| 100 | 12,755 | 2 | 2,798 |
| 27 | 8,538 | 1 | 2,762 |
| 6 | 6,380 | 2 | 2,744 |
| 2 | 6,117 | 2 | 2,678 |
| 6 | 5,644 | 1 | 2,631 |
| 12 | 5,491 | 1 | 2,570 |
| 56 | 5,096 | 1 | 2,534 |
| 17 | 4,874 | 1 | 2,514 |
| 10 | 4,486 | 1 | 2,454 |
| 15 | 4,244 | 1 | 2,419 |
| 7 | 4,418 | 1 | 2,346 |
| 1 | 4,137 | 1 | 2,308 |
| 5 | 4,001 | 1 | 2,256 |
| 3 | 3,949 | 1 | 2,232 |
| 5 | 3,828 | 1 | 2,166 |
| 3 | 3,585 | 1 | 2,008 |
| 7 | 3,515 | 2 | 1,953 |
| 3 | 3,427 | 1 | 1,691 |
| 3 | 3,341 | 1 | |
| 3 | 3,310 | | |
| 3 | 3,170 | | |
| 4 | 3,156 | | |
| 4 | 3,149 | | |
| 1 | 3,064 | | |
| 2 | 2,979 | | |
| 1 | 2,944 | | |
| 1 | 2,903 | | |

2. Process for the preparation of a crystalline modification of the hydrogen chloride of the 2-dimethylaminoethyl ester of n-butylaminobenzoic acid according to Claim 1, characterized in that a solution of the hydrogen chloride of the 2-dimethylaminoethyl ester of n-butylaminobenzoic acid in polymorphic form in water or an organic solvent or in a mixture thereof is cooled by means of a refrigerant at a rate of not less than 3°C/min until complete crystallization thereof, with subsequent removal of the crystals obtained and drying thereof.

3. Process according to Claim 2, characterized in that the organic solvent used is ethanol.

4. Process according to Claim 2, characterized in that the refrigerant used is liquid nitrogen, while the drying is carried out by evacuation to a pressure of not more than $10^{-2}$ mmHg.

5. Drug for anaesthesia of the eyes which contains an active substance and a pharmaceutical solvent, characterized in that it contains the crystalline modification of the hydrogen chloride of the 2-dimethylaminoethyl ester of n-butylaminobenzoic acid according to Claim 1 in an amount of 0.05 to 0.5% by weight as the active substance.

6. Drug according to Claim 5, characterized in that it additionally contains alkylcellulose in an amount of 0.1 to 0.75% by weight.

7. Drug according to Claim 6, characterized in that it contains water-soluble methylcellulose as the alkylcellulose.

**Revendications**

1. Cristal modifié de chlorhydrate de n-butylaminobenzoate de 2-diméthylaminoéthyle, caractérisé par une fusion à une température de 148,6 ± 0,3°C avec un seul effet endothermique et par les écartements des plans réticulaires et les intensités de réflexion relatives I suivants :

| I | d,Å | I | d,Å |
|---|---|---|---|
| 3 | 26,140 | 2 | 2,979 |
| 100 | 12,755 | 1 | 2,944 |
| 27 | 8,538 | 1 | 2,903 |
| 6 | 6,380 | 4 | 2,824 |
| 2 | 6,117 | 2 | 2,798 |
| 6 | 5,644 | 1 | 2,762 |
| 12 | 5,491 | 2 | 2,744 |
| 56 | 5,096 | 2 | 2,678 |
| 17 | 4,874 | 1 | 2,631 |
| 10 | 4,486 | 1 | 2,570 |
| 15 | 4,244 | 1 | 2,534 |
| 7 | 4,418 | 1 | 2,514 |
| 1 | 4,137 | 1 | 2,454 |
| 5 | 4,001 | 1 | 2,419 |
| 3 | 3,949 | 1 | 2,346 |
| 5 | 3,828 | 1 | 2,308 |
| 3 | 3,585 | 1 | 2,256 |
| 7 | 3,515 | 2 | 1,232 |
| 3 | 3,427 | 1 | 1,166 |
| 3 | 3,341 | 1 | 2,008 |
| 3 | 3,310 | 2 | 1,953 |
| 3 | 3,170 | 1 | 1,691 |
| 4 | 3,156 | 1 | |
| 4 | 3,149 | | |
| 1 | 3,064 | | |

2. Procédé de préparation d'un cristal modifié de chlorhydrate de n-butylaminobenzoate de 2-diméthylaminoéthyle, selon la revendication 1, caractérisé en ce que l'on refroidit une solution de chlorhydrate de n-butylaminobenzoate de 2-diméthylaminoéthyle, sous forme polymorphe dans de l'eau ou dans un solvant organique ou dans un mélange de ceux-ci, avec des moyens de refroidissement, à une vitesse non inférieure à 8 °C/min jusqu'à la cristallisation complète dudit composé, en séparant ensuite les cristaux obtenus et en les séchant.

3. Procédé selon la revendication 2, caractérisé en ce que l'on utilise de l'éthanol comme solvant organique.

4. Procédé selon la revendication 2, caractérisé en ce que l'on utilise de l'azote liquide comme moyen de refroidissement, et en ce que l'on effectue le séchage sous vide à une pression d'au plus $10^{-2}$ Torr.

5. Préparation pharmaceutique pour l'anesthésie oculaire, qui comprend un ingrédient actif et un solvant pharmaceutique, caractérisée en ce qu'elle contient en tant qu'ingrédient actif un cristal modifié de chlorhydrate de n-butylaminobenzoate de 2-diméthylaminoéthyle, selon la revendication 1, à raison de 0,05 à 0,5 % en poids.

6. Préparation pharmaceutique selon la revendication 5, caractérisée en ce qu'elle comprend en outre une alkylcellulose à raison de 0,1 à 0,75 % en poids.

7. Préparation pharmaceutique selon la revendication 6, caractérisée en ce qu'elle comprend comme alkylcellulose de la méthylcellulose hydrosoluble.